# FASCICULE DE BREVET EUROPEEN

(11) **EP 0 815 826 B1**
(45) Date de publication et mention de la délivrance du brevet: **26.03.2008**
(21) Numéro de dépôt: 97401269.2
(22) Date de dépôt: 05.06.1997
(51) Int. Cl.: A61K 8/00, A61Q 3/02

(54) **Nouvelles compositions cosmétiques comprenant des agents de coloration cristaux liquides et leur utilisation**
Kosmetische zusammensetzungen enthaltend flüssigkristalline Färbemittel und ihre Verwendung
Cosmetic compositions comprising liquid crystal colouring agents and their use

(30) Priorité: 02.07.1996 FR 9608221
(43) Date de publication de la demande: 07.01.1998
(73) Titulaire: L'ORÉAL, 75008 Paris (FR)
(72) Inventeur: Lemmann, Patricia, 92320 Chatillon (FR); Mellul, Patricia, 94240 L'Hay Les Roses (FR); Collette, Annick, 94600 Choisy Le Roi (FR); Bara, Isabelle, 75013 Paris (FR)
(74) Mandataire: Miszputen, Laurent

(56) Documents cités:
- EP-A- 0 686 674
- WO-A-90/02161
- DE-A- 4 240 743
- DE-A- 19 629 761
- GB-A- 2 282 145
- US-A- 4 301 023
- J.-P. CAQUET ET AL: "Les cristaux liquides dans les cosmétiques" PARFUMS COSMETIQUES AROMES, no. 91, 1990, pages 77-83, XP000135628
- DATABASE WPI Week 9024 Derwent Publications Ltd., London, GB; AN 90-181522 XP002028850 "Liquid crystal compsn. for cosmetics - contains cholesteryl oleate, acetate and further fatty ester for colour development at room temp." & JP 02 117 987 A (NISSHIN OIL MILLS) , 2 mai 1990
- DATABASE WPI Week 9042 Derwent Publications Ltd., London, GB; AN 90-315971 XP002028851 "Cosmetic material - contg. liq. crystal with good colour development, mildness to skin, and improved water-retaining ability" & JP 02 223 506 A (MIKIMOTO SEIYAKU) , 5 septembre 1990

## Description

La présente invention concerne une nouvelle composition cosmétique, en particulier une composition cosmétique de maquillage destinée au maquillage des ongles, comprenant un agent de coloration de type cristaux liquides (ci-après agent de coloration CL), permettant d'obtenir des effets de coloration nouveaux, en particulier d'obtenir des couleurs distinctes selon l'incidence de la lumière et l'angle d'observation.

Les compositions de maquillage, tels que les poudres libres ou compactées, les fonds de teint, les fards à joues, les fards à paupières, les rouges à lèvres ou les vernis à ongles, sont constitués d'un véhicule aproprié et de différents agents de coloration destinés à conférer une certaine couleur aux compositions avant et/ou après leur application sur la peau, les muqueuses (en particulier les lèvres) ou les phanères.

Pour créer des couleurs, on utillise aujourd'hui une gamme d'agents de coloration assez limitée, en particuliers de pigments tels que des laques, des pigments minéraux ou des pigments nacrés.

Les laques permettent d'obtenir des couleurs vives, mais pour la plupart sont instables à la lumière, à la température et au pH. Certaines présentent également l'inconvénient de tacher la peau de manière disgracieuse après application, par dégorgement du colorant.

Les pigments minéraux, en particulier les oxydes minéraux sont au contraire très stables, mais donnent des couleurs plutôt ternes et pâles.

Pour obtenir des effets colorés, ont peut employer des pigments nacrés de couleurs variées, mais jamais intenses, qui permettent d'obtenir des effets irrisés mais le plus souvent assez faibles.

La présente invention concerne donc une nouvelle composition cosmétique comprenant un support cosmétiquement acceptable et un agent de coloration CL susceptible de produire une couleur comprise dans une gamme de nuances comprises entre au moins deux couleurs spécifiques et variant en fonction de l'incidence de la lumière et de l'angle d'observation; ledit agent de coloration étant choisi parmi les polymères linéaires ou cycliques sur lesquels sont greffés des groupes mésomorphes et que ledit agent de coloration CL est utilisé en combinaison avec au moins un agent de coloration non CL, est dans laquelle la quantité totale d'agents de coloration, CL et non CL, étant comprise entre 0,1 et 30% en poids par rapport au poids total de la composition et le rapport pondéral agents de coloration CL / agents de coloration non CL étant compris entre 20/1 et 1/5.

Par couleur, on entend de préférence selon l'invention toute couleur du spectre visible. De préférence, les couleurs spécifiques des agents de coloration sont émises dans la lumière visible.

Par agent de coloration on entend un matériau destiné à donner à une matière ou composition une coloration durable. On peut distinguer d'une part les colorants essentiellement solubles dans leur milieu d'utilisation et d'autres part les pigments constitués de fines particules qui, par opposition aux colorants, sont insolubles dans leur milieu d'utilisation.

Ces agents de coloration CL sont en particulier décrits dans les brevets et demandes de brevet EP29 162, EP 66 137, EP 60 335, DE 37 32 115, EP 333 022, EP 358 208, EP 385 376, EP 404 140, EP 424 259, EP 431 466, EP 446 912, EP 446 183, EP 545 409, WO 94/09086, DE 43 28 761, EP 635 749, EP661 287, EP 709 445, JP 60 148 173, JP 07 278 308, US 5 364 557, GB 2 280 681, GB 2 282 145, GB 2 276 883, GB 2 282 146, WO 95/32247, WO 95/32248, EP 601 483, EP 626 386, EP 686 674, EP 711 780.

EP 0 686 674 A1 et EP 0601 483 A1 décrivent des combinaisons d'agents de coloration CL et non CL.

Les agents de coloration CL sont plus particulièrement des silicones ou des éthers de cellulose, sur lesquels sont greffés des groupes mésomorphes, peuvent être employés seuls et/ou enrobés sur des supports inertes tels que des micas, et/ou associés avec d'autres agents de coloration non CL.

Les groupes mésomorphes sont généralement des groupes de formule

-D-(X¹ₐ-A¹_{b}-A²_{c})_{d}-Zₑ-(-X²_{f}-A³_{g}-A⁴ₕ)ᵢ-A⁵ₖ

dans laquelle
D représente un reste alkylène en C₁-C₂₀, éventuellement substitué par un ou plusieurs halogènes, pour lequel on peut remplacer une ou plusieurs des unités méthylène non voisines par un groupe X¹,
X¹ et X² représentent indépendemment des radicaux divalents -O-, -COO-,-CONH-, -CO-, -S-, -C≡C-, -CH=CH-, -CH₂-CH₂-, -CH=N-, -N=N- ou -N=N(O)-,
A¹, A², A³ et A⁴, représentent indépendament des radicaux divalents 1,4-phénylène, 1,4-cyclohexylène, arylènes, hétéroarylènes, cycloalkylènes éventuellement substitués,
Z représente indépendamment des radicaux divalents à tétravalents, benzène-1,4 cyclohexane ou benzène-1,3-cyclopentane,
A⁵ représente indépendamment un radical alkyle, alkoxy ou cycloalkyle, saturés ou insaturés, ayant 1 à 16 atomes de carbone, un radical stéroïdien, un halogène, unatome d'hydrogène, un radical hydroxyle, nitrile ou trialkylsilyloxy,
a, b, c, d, f, g, h, i et k représentent indépendamment un entier compris entre 0 et 3,
e représente 0 ou 1,
avec la somme a+b+c+d+e+f+g+h+i+k étant suppérieure ou égale à 2, et la somme d+i étant inférieure ou égale à 4,
étant entendu que ce groupe mésomorphe ne comprend pas de radical peroxyde.

En particulier, les agents de coloration CL de l'invention peuvent se présenter sous forme de poudres blanches amorphes et être assimilés à des « pigments ». La couleur et/ou l'effet de coloration n'apparait qu'à l'étalement de la composition qui les contient, notament en fonction de la couleur du support sur lequel on l'étale et/ou de la présence d'éventuels agents de coloration non CL associés.

Les « pigments » CL peuvent notamment être constitués de substances orientées à réticulation tridimentionelle comprenant un groupe mésomorphe, en particulier tel que défini ci-dessus, et pouvant comprendre au moins un groupe polymérisable, ayant une épaisseur moyenne comprise entre 1 et 100 µm et un diamètre moyen compris entre 1 et 10 000 µm. Ces pigments CL à structure mésomorphe peuvent être préparés par réticulation après orientation, après l'ajout éventuel de pigments non CL, puis broyage à la granulométrie souhaitée.

Une forme particulèrement préférée d'agent de coloration CL conforme à l'invention consiste en des polyorganosiloxanes cycliques greffés par des groupements cholestériques et biphényliques. Ils sont décrits notamment dans l'article de H.J. EBERLE, A MILLER, F. H. KREUZER Liquid Crystals, 1989, Vol 5, N°3, 907-916, dans l'article de J. PINSL, CHR. BRAÜCHIE, F. H. KREUZER, Journal of Molecular Electronics, Vol 39-13 (1987) et le brevet USP 4 410 570.

Ils sont choisis encore plus particulièrement parmi les cyclométhicones greffées par des groupements cholestériques et biphényliques de formule sui vante : 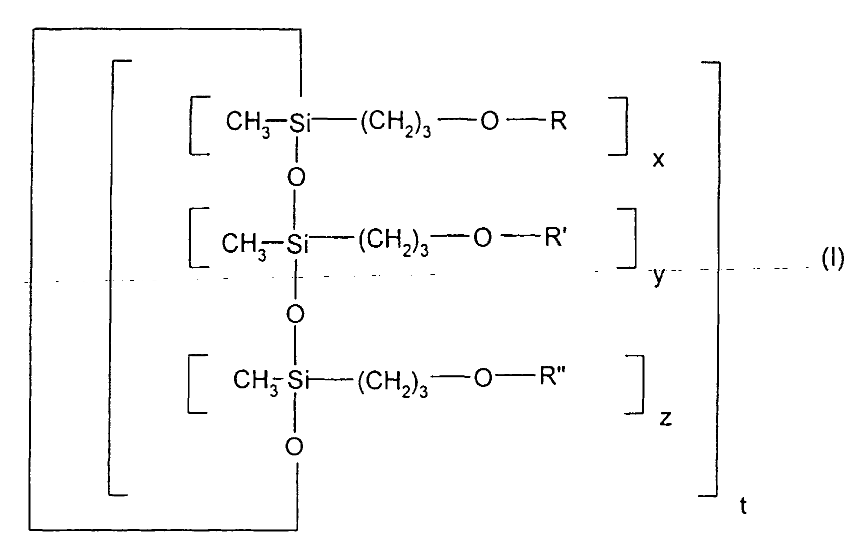 dans laquelle :
0 ≤ x ≤ 1 (de préférence 1) ; 0 ≤ y ≤ 1 (de préférence 1) ; 0 ≤ z ≤ 1 (de préférence 1) avec x + y +z # 0 ; 3 ≤ t ≤ 10 ;
R désigne un groupe de formule suivante : 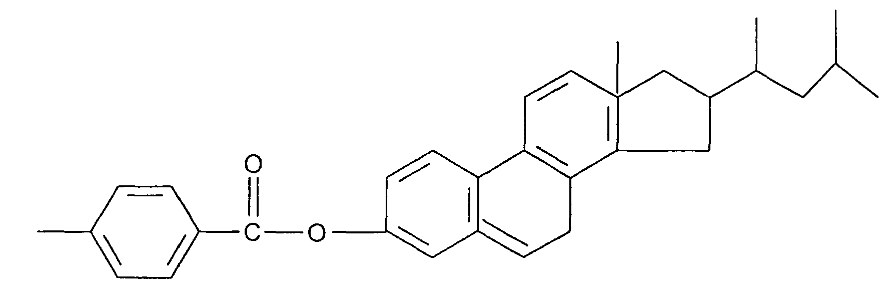
R' désigne un groupe de formule suivante : 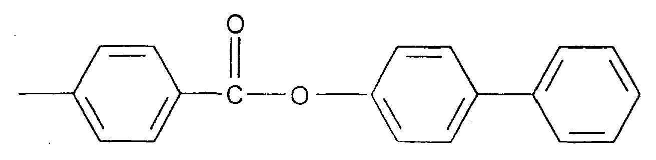
R" désigne un groupe de formule suivante

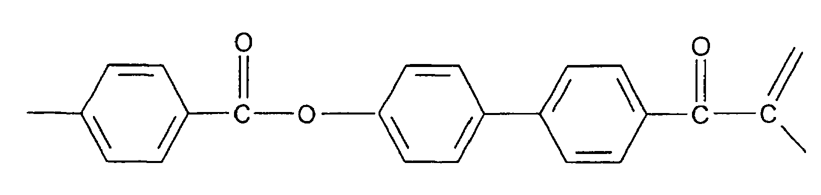

Ces composés se présentent généralement sous forme de poudres branches amorphes. La couleur et/ou l'effet de coloration sont compris dans une gamme de nuances comprises entre au moins deux couleurs spécifiques et varie en fonction de l'incidence de la lumière et de l'angle d'observation. La couleur et/ou l'effet de coloration n'apparaissent qu'à l'étalement de la composition qui les contient, notament en fonction de la couleur du support sur lequel on l'étale et de la présence d'agents de coloration non CL associés.

A titre d'exemples d'agent de coloration CL répondant à cette définition, on peut citer en particulier les « pigments CL » commercialisés par la société WACKER sous les dénominations SLM 41101 (BLEU/VERT), SLM 41102 (ROUGE/OR) et SLM 41103 (JAUNE/VERT).

Les compositions cosmétiques selon l'invention sont celles concernant le maquillage des ongles notamment les vernis à ongles anhydres et aqueux.

Par agent de coloration non CL, on entend des colorants non CL et/ou des pigments non CL usuels de la technique

Les pigments sont des substances naturelles ou de synthèse constituées de fines particules qui, par opposition aux colorants, sont insolubles dans leur milieu d'utilisation, dont la fonction principale consiste à donner une coloration. On distingue différents types de pigments, les pigments minéraux, les pigments organiques, les laques ou les pigments nacrés. Les laques sont des colorants adsorbés sur des particules insolubles, l'ensemble restant essentiellement insoluble dans le milieu d'utilisation. Les pigments nacrés sont des substances naturels ou synthétiques, qui diffractent et réfléchissent la lumière pour donner un effet irisé ou brillant.

Parmi les colorants, on peut citer les colorants organiques naturels, tels que le carmin de cochenille (Cl 75 470), ou de synthèse, tels que les colorants halogéno-acides, azoïques, anthraquinoniques. On peut également citer des colorants minéraux tels que le sulfate de cuivre.

Parmi les pigments minéraux, on peut citer les oxydes métalliques, en particulier les oxydes de zirconium, de cérium, de zinc ou de chrome (Cl 77 288), le dioxyde de titane (Cl 77 891), les oxydes de fer noir, jaune, rouge et brun (Cl 77 499, Cl 77492, Cl 77491), le violet de manganèse (Cl 77 742), le bleu outremer (Cl 77 007), le bleu ferrique (Cl 77 510), l'hydrate de chrome (Cl 77 289), la poudre d'argent ou la poudre d'aluminium.

Parmi les pigments organiques, on peut citer le noir de carbone (Cl 77 266), ou le D & C Rouge 36.

Les laques sont généralement constituées par des sels métalliques (notamment Al, Zr, Ca, Na) de colorants organiques, adsorbés sur des particules, par exemple d'alumine, de sulfate de baryum, de colophane, etc. Parmi les laques, on peut citer celles connues sous les dénominations: D & C Red 21 (Cl 45 380), D & C Orange 5 (Cl 45 370), D & C Red 27 (Cl 45 410), D & C Orange 10 (Cl 45 425), D & C Red 3 (Cl 45 430), D & C Red 7 (Cl 15 850:1), D & C Red 4 (Cl 15 510), D & C Red 33 (Cl 17 200), D & C Yellow 5 (Cl 19 140), D & C Yellow 6 (Cl 15 985), D & C Green 5 (Cl 61 570), D & C Yellow 10 (Cl 77 002), D & C Green 3 (Cl 42 053), D & C Blue 1 (Cl 42 090).

Parmi les pigments nacrés, on peut citer l'oxychlorure de bismuth, le mica recouvert d'oxyde de titane, d'oxyde de fer, ou de pigments naturels, par exemple le mica titane coloré.

Dans les compositions selon l'invention, la quantité totale d'agents de coloration, CL et non CL, est comprise entre 0,1 et 30 % en poids par rapport au poids total de la composition, en particulier entre 1 et 20 % en poids.

Le rapport pondéral agents de coloration CL / agents de coloration non CL est compris entre 20 / 1 et 1 / 5, de préférence compris entre 10/1 et 1/5, plus préférentiellement compris entre 5/1 et 1/5.

Les compositions cosmétiques selon l'invention peuvent en outre contenir des charges additionnelles usuelles en cosmétique.

Les charges sont des matériaux naturels ou synthétiques dont la fonction principale consiste à modifier les propriétés physico-chimiques (rhéologiques, mécaniques, optiques) et/ou cosmétiques d'une composition. Les charges sont incolores ou plus ou moins blanches à l'état sec. Elles sont presque transparentes lorsque dispersées dans un liant.

Parmi les charges, on peut citer le talc qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ; les micas qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm; les micas peuvent être d'origine naturelle (par exemple muscovite, margarite, roscoelithe, lépidolithe, biotite) ou d'origine synthétique; ils sont généralement transparents et permettent de conférer à la peau un aspect satiné ; l'amidon, en particulier l'amidon de riz ;la silice; le kaolin, qui est un silicate d'aluminium hydraté, qui se présente sous la forme de particules de forme isotrope ayant des dimensions généralement inférieures à 30 µm, et qui possède de bonnes propriétés d'absorption des corps gras ;les poudres de Nylon^{®} (Orgasol notamment) et de polyéthylène; le Téflon^{®}; le nitrure de bore; des microsphères de copolymères, telles que l'Expancel^{®} (Nobel Industrie), le polytrap^{®} (Dow Corning) et les microbilles de résine de silicone (Topsearls^{®} de Toshiba, par exemple); le carbonate de caCLium précipité qui, sous forme de particules de dimensions inférieures à 10 µm, a un toucher onctueux et permet d'obtenir un aspect mat ; le carbonate ou l'hydrocarbonate de magnésium, qui possèdent notamment des propriétés de fixation des parfums ; des savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, etc; ces savons, présents généralement sous la forme de particules ayant des dimensions inférieures à 10 µm ont un toucher onctueux et facilitent l'adhérence de la poudre sur la peau

Selon le type de formulation, les charges peuvent représenter de 0,01 à 90 % en poids de la composition.

Enfin, lorsque le maquillage de base est noir, le noir disparait et seules les couleurs spécifiques de l'agent de coloration CL sont révélées.

Le film de maquillage obtenu après application présente des couleurs distinctes selon l'orientation de la lumière incidente et de l'angle d'observation. On peut obtenir par exemple une couleur dans la gamme correspondant au couple bleu/vert ou rouge/vert, selon l'agent de coloration CL employé, et la couleur spécifique du maquillage de base. Ces effets de couleurs s'accompagnent d'un effet de scintillement très lumineux. Dans certains cas, on peut même distinguer des nuances intermédiaires.

Ainsi, lorsque l'on emploie un agent de coloration CL dont l'une des couleurs du couple de couleurs spécifiques est essentiellement similaire à la couleur de l'agent de coloration non CL (ou de l'association d'agents de coloration non CL) présents dans la composition selon l'invention, l'agent de coloration CL vient renforcer et intensifier ladite couleur, tout en produisant un changement de couleur selon l'incidence de la lumière et l'angle d'observation.

Par contre, lorsque l'on emploie un agent de coloration CL dont les couleurs spécifiques sont différentes de la couleur de l'agent de coloration non CL (ou de l'association d'agents de coloration non CL) présents dans la composition selon l'invention, on obtient un nouveau ton de la couleur de l'agent de coloration non CL (ou de l'association d'agents de coloration non CL), plus ou moins intense selon la saturation de ladite couleur. Plus la couleur considérée est saturée, plus la modification de sa tonalité sera intense.

Enfin, lorsque l'on emploie un agent de coloration CL avec un agent de coloration noir, ce sont les couleurs spécifiques de l'agent de coloration CL qui sont révélées, le noir disparaissant.

La présente invention concerne donc une composition cosmétique comprenant à titre d'agent de coloration, la combinaison d'un agent de coloration CL tel que défini ci-dessus susceptible de produire une couleur dans une gamme de nuances correspondant à au moins deux couleurs, et au moins un agent de coloration non CL dont la couleur est essentiellement similaire à l'une desdites couleurs spécifiques de l'agent de coloration CL. Dans ce cas, l'agent de coloration CL remplit la fonction d'agent de renfort de couleur

La présente invention concerne également une composition cosmétique comprenant à titre d'agent de coloration, la combinaison d'un agent de coloration CL tel que défini ci-dessus susceptible de produire une couleur dans une gamme de nuances comprises entre au moins deux couleurs spécifiques, et au moins un agent de coloration non CL dont la couleur est différente desdites couleurs spécifiques de l'agent de coloration CL. Dans ce cas, l'agent de coloration CL remplit la fonction d'agent modulateur de couleur.

Par agent d'uniformisation du teint on entend selon l'invention un composé (ou une composition) dont la fonction consiste à réduire et/ou corriger les imperfections coloristiques de la peau, telles que les taches de couleurs, la couperose, les boutons, etc.

Par agent de renfort de couleur on entend selon l'invention un composé (ou une composition) dont la fonction consiste à saturer la couleur en question.

Par agent modulateur de couleur on entend selon l'invention un composé (ou une composition) dont la fonction consiste à modifier la tonalité de ladite couleur (effectuer un déplacement de ton). Par exemple, pour une couleur rouge de ton bleuté, on obtient avec un modulateur de couleur approprié une couleur rouge de ton orangé.

Par agent de coloration non CL on entend selon la présente invention tout agent de coloration usuel non CL employé dans des compositions cosmétiques. Lorsque la couleur de la composition correspond à un mélange de plusieurs agents de coloration non CL, l'expression "agent de coloration non CL" englobe également lesdits mélanges d'agents de coloration non CL. Dans ce cas, les expressions "couleur essentiellement similaire à l'une des couleurs spécifiques de l'agent de coloration CL" ou "couleur différente des couleurs spécifiques de l'agent de coloration CL" s'appliquent à la couleur dudit mélange d'agents de coloration non CL.

Les compositions selon la présente invention peuvent notamment se présenter sous forme d'émulsion huile-dans-l'eau ou eau-dans-l'huile, ou sous forme d'une suspension en milieu solvant, ou encore sous forme de poudre libre, de poudre compactée, ou d'un solide ou d'une pâte anhydre, ou encore sous forme de gel ou de mousse. Les modes opératoires pour la préparation de ces différents types de composition sont bien connus de l'homme du métier.

Lorsqu'elles sont utilisées sous forme d'émulsion, les compositions selon l'invention peuvent contenir des agents tensio-actifs bien connus dans l'état de la technique. Ces tensio-actifs peuvent constituer de 0,01 à 30 % en poids par rapport au poids total de la composition.

Une réalisation particulièrement préférée consiste à préparer des émulsions anioniques ou non-ioniques en utilisant des agents tensio-actifs anioniques ou non-ioniques dans des proportions de préférence comprises entre 2 et 30 % en poids par rapport au poids total de la composition.

Parmi les agents tensio-actifs anioniques qui peuvent être utilisés seuls ou en mélange, on peut citer en particulier les sels alcalins, les sels d'ammonium, les sels d'amines ou les sels d'aminoalcools des composés suivants :
- les alcoylsulfates, alcoyléther sulfates, alcoylamides sulfates et éthers sulfates, alcoylarylpolyéthersulfates, monoglycérides sulfates,
- les alcoylsulfonates, alcoylamides sulfonates, alcoylarylsulfonates, α-oléfines sulfonates, paraffines sulfonates,
- les alcoylsulfosuccinates, alcoyléthersulfosuccinates, alcoylamides sulfosuccinates,
- les alcoylsulfosuccinamates,
- les alcoylsulfoacétates, les alcoylpolyglycérol carboxylates,
- les alcoylphosphates/alcoylétherphosphates,
- les acylsarcosinates, alcoylpolypeptidates, alcoylamidopolypeptidates, acyliséthionates, alcoyllaurates.

Le radical alcoyle ou acyle dans tous ces composés désigne généralement une chaîne de 12 à 18 atomes de carbone.

D'autres agents tensio-actifs anioniques sont constitués par les sels d'acides gras tels que les acides oléique, ricinoléique, palmitique, stéarique, les acides d'huile de coprah ou d'huile de coprah hydrogénée et notamment les sels d'amines tels que les stéarates d'amines.

On peut également citer :
- les acyl lactylates dont le radical acyle comprend de 8 à 20 atomes de carbone,
- les acides carboxyliques d'éthers polyglycoliques répondant à la formule :

   Alk-(OCH₂-CH₂)ₙ-OCH₂-COOH

   sous forme acide ou salifiée où le substituant Alk correspond à une chaîne linéaire ayant de 12 à 18 atomes de carbone et où n est un nombre entier compris entre 5 et 15.

Parmi les tensio-actifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone. On peut également citer des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose.

D'autres composés entrant dans cette Icasse sont les produits de condensation d'un α-diol, d'un monoalcool, d'un alcoylphénol, d'un amide ou d'un diglycolamide avec le glycidol ou un précurseur de glycidol.

Les tensio-actifs non-ioniques principalement utilisés sont des alcools polyéthoxylés ou polyglycérolés tels que les alcools stéarylique, cétylstéarylique ou oléique polyéthoxylés.

Les tensio-actifs anioniques préférentiellement utilisés sont des stéarates d'amines.

Les compositions selon l'invention peuvent également se présenter sous forme d'un gel, d'une solution aqueuse ou hydroalcoolique d'un ou plusieurs polymères hydrosolubles tels que les dérivés de l'acide polyacrylique ou sous forme de gels émulsionnés obtenus par dispersion d'huiles dans des gels à l'aide d'émulsionnants tels que les Pemulens^{®} de la Société GOODRICH.

Les compositions selon la présente invention peuvent en outre contenir des ingrédients usuels choisis parmi des adoucissants, des conservateurs, des séquestrants, des parfums, des épaississants, des agents de cohésion, des polymères ainsi que des agents alcalinisants ou acidifiants, des hydratants et des actifs hydrosolubles.

Les épaississants utilisables peuvent être naturels ou synthétiques. Parmi les épaississants naturels, on peut citer les gommes de diverses sortes telles que les gommes arabique, de guar ou de caroube. Parmi les épaississants de synthèse, on peut citer les dérivés cellulosiques comme l'hydroxyéthylcellulose, la carboxyméthylcellulose, les dérivés de l'amidon, les dérivés d'éthers de cellulose possédant des groupes ammonium quaternaires, les polysaccharides cationiques, les sels de polymères acryliques ou méthacryliques, les polyènes ou les polysiloxanes.

On peut également obtenir un épaississement des compositions par mélange de polyéthylèneglycol et de stéarate et/ou de distéarate de polyéthylèneglycol ou par un mélange d'esters phosphoriques et d'amides gras.

Selon l'invention la phase huileuse peut représenter de 0,1 à 50 % en poids par rapport au poids total de l'émulsion.

Elle peut être constituée d'huiles, et/ou de cires.

Les cires et les huiles peuvent être d'origine végétale, animale, minérale ou synthétique.

Parmi les huiles végétales on peut citer l'huile de jojoba, l'huile d'olive, l'huile d'amande douce, l'huile d'avocat, l'huile de coco, l'huile de germe de blé, l'huile de maïs, l'huile de palme, l'huile de sésame, l'huile de soja, l'huile d'argan, l'huile d'onagre, l'huile de bourrache et les huiles essentielles.

Parmi les huiles animales on peut notamment citer l'huile de poisson.

Parmi les huiles minérales on peut citer l'huile de vaseline et d'isohexadécane.

Parmi les huiles synthétiques on peut mentionner les palmitates d'éthyle et d'isopropyle, le palmitate d'éthyl-2 hexyle, les myristates d'alkyle tels que le myristate d'isopropyle, de butyle, de cétyle, le stéarate d'hexyle, les triglycérides des acides octanoïque et décanoïque, le ricinoléate de cétyle et l'octanoate de stéaryle, les huiles de silicone, les huiles perfluorées, les huiles de silicone fluorées.

La phase huileuse peut par ailleurs contenir des colorants, des filtres solaires, des antioxydants, des conservateurs et des principes actifs lipophiles.

Selon l'invention les compositions anhydres qui peuvent se présenter sous forme de poudre libre ou compactée, de fard solide, pâteux ou liquide peuvent contenir un liant qui peut représenter de préférence de 0,01 à 95 % en poids par rapport au poids total de la composition.

Parmi les agents liants, on peut citer notamment les huiles animales, végétales ou synthétiques, les mélanges d'huile(s) et de cire(s) et en particulier l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, etc. ; des huiles d'hydrocarbures, telles que des huiles de paraffine, le squalane, la vaseline, etc. ; des esters tels que le myristate d'isopropyle, le palmitate d'isopropyle, le stéarate de butyle, le laurate d'hexyle, l'isononanate d'isononyle, le palmitate de 2-éthyl hexyle, le laurate de 2-hexyl-décyle, le palmitate de 2-octyldécyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine, etc. ; des huiles de silicone comme les polyméthylsiloxanes, les poly-méthylphénylsiloxanes, des polysiloxanes modifiés par des acides gras, des polysiloxanes modifiés par des alcools gras, des polysiloxanes modifiés par des polyoxyalkylènes, des silicones fluorées, etc. ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique, l'acide isostéarique, etc. ; des alcools gras supérieurs tels que le cétanol, l'alcool stéarylique, l'alcool oléique, etc. ; les cires peuvent être choisies notamment parmi la cire de carnauba, la cire de candelilla, la cire d'abeille, la cire de baleine, des lanolines, des cires microcristallines, etc.

Le liant peut contenir en outre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa).

Parmi les huiles volatiles pouvant être présentes comme agents d'étalement dans la composition de l'invention, on citera par exemple les huiles de silicone telles que l'hexaméthyldisiloxane, le cyclopentadiméthylsiloxane, le cyclotétraméthylsiloxane, des huiles fluorées comme celle commercialisée sous la dénomination GALDEN^{®} (MONTEFLUOS) ou des huiles isoparaffiniques telles que celles commercialisées sous la dénomination ISOPAR® (E, G, L ou H ; EXXON CHEMICAL).

Comme mentionné ci-dessus, les compositions selon l'invention peuvent également se présenter sous forme d'un vernis à ongles anhydre ou aqueux.

Lorsque les compositions se présentent sous forme d'un vernis à ongles anhydre, le système solvant représente environ de 55 % à 90 % en poids par rapport au poids total du vernis.

Ce système solvant est constitué par un mélange de divers solvants organiques volatils tels que l'acétone, l'acétate d'éthyle, l'acétate de butyle, l'acétate de méthoxy-2 éthyle, la méthyléthylcétone, la méthylisobutylcétone, l'acétate de méthyle, l'acétate d'amyle et l'acétate d'isopropyle.

Le système solvant peut également comprendre un diluant tel que l'hexane ou l'octane ou encore un hydrocarbure aromatique tel que le toluène ou le xylène en une proportion de 10 à 35 % en poids par rapport au poids total du vernis.

La matière filmogène du vernis est généralement présente à une concentration comprise entre 5 et 20 % en poids par rapport au poids total du vernis.

Parmi les matières filmogènes on peut notamment citer les nitrocelluloses du type "RS" ou "SS" et en particulier la nitrocellulose type 1/4"RS", la nitrocellulose type 1/2"RS", la nitrocellulose type 1/2"SS" et la nitrocellulose type 3/4"RS".

Les vernis contiennent également un agent plastifiant généralement présent à une concentration comprise entre 2 et 10 % en poids par rapport au poids total du vernis. Parmi ceux-ci on peut notamment mentionner le phosphate de tricrésyle, le benzoate de benzyle, le citrate de triéthyle, le citrate de tributyle, l'acétylcitrate de triéthyle, l'acétylcitrate de triétyl-2 hexyle, le phtalate de diamyle, le camphre.

Les vernis selon l'invention contiennent également une résine généralement présente à une concentration comprise entre 0,5 et 15 % en poids par rapport au poids total du vernis.

Parmi les nombreuses résines utilisables on peut notamment citer les résines du type aryl-sulfonamide formaldéhyde ou aryl-sulfonamide époxy notamment les résines connues sous les dénominations commerciales SANTOLITE MHP^{®} et SANTOLITE MS 80 %^{®}.

Lorsque les vernis à ongles se présentent sous forme aqueuse, ils contiennent une dispersion d'une substance filmogène synthétique dans laquelle divers additifs usuels peuvent être ajoutés tels qu'une matière filmogène, un épaississant, un régulateur de pH, un réticulant, un anti-mousse, etc.

Comme dispersion aqueuse synthétique, on peut, entre autre, utiliser des dispersions d'acétate de polyvinyle, de polyuréthanne, de polymères ou copolymères acryliques et de copolymères d'acétate de polyvinyle.

Selon l'invention, la dispersion aqueuse synthétique représente environ de 10 à 80 % en poids du vernis.

La matière filmogène est généralement présente à une concentration comprise entre 5 et 20 % en poids par rapport au poids total du vernis.

Parmi les matières filmogènes on peut notamment citer les dérivés de cellulose solubles dans l'eau.

Les vernis selon l'invention peuvent également contenir une résine généralement présente à une concentration comprise entre 0,5 et 15 % en poids par rapport au poids total du vernis.

Parmi les résines utilisables on peut notamment citer les résines du type acrylique, styrénique, acrylate-styrénique et vinylique.

Les vernis à ongles anhydres ou aqueux selon l'invention peuvent également contenir des adjuvants couramment utilisés dans les vernis à ongles tels que par exemple des filtres U.V.

Les exemples ci-après permettent d'illustrer les différents effets de couleurs obtenus avec différents pigments CL dans les compositions selon l'invention. Les pourcentages des constituants des compositions sont exprimés en poids, la somme de tous les constituants étant égale à 100.

### Exemple 1 Vernis à ongles incolore (il ne fait pas partie de l'invention)

| Vernis à ongle aqueux | |
|---|---|
| Composition | % |
| Dispersion aqueuse de polyuréthane Extrait sec: 34% Sancure | 94,50 |
| Agent d'étalement | 0,50 |
| Pigment CL | 5,00 |

| Vernis à ongles anhydre | |
|---|---|
| Composition | % |
| Nitrocellulose | 10,820 |
| Résine toluène sulfonamide formaldéhyde "KET JENFLEX MS 80" AKZO | 10,740 |
| Acétylcitrate de tributyle "Citroflex A4" PFIZER | 6,495 |
| Toluène | 30,910 |
| Acétate de butyle | 20,640 |
| Acétate d'éthyle | 9,270 |
| Isopropanol | 7,720 |
| Pigment CL | 2,000 |
| Acide citrique | 0,055 |

Les vernis obtenu est blanc irisé dans le flacon.

En applicant directement les vernis à ongles selon l'invention sur l'ongle, on obtient un léger effet coloré dans les tons du couple de couleur du pigment employé.

En appliquant le vernis à ongle selon l'invention avec le pigment SLM 41101, sur une base de vernis noir, on obtient une couleur intense qui passe du bleu au vert selon l'angle d'observation (ou l'inlcinaison de l'ongle). On retrouve le même effet pour les couleurs spécifiques des pigments SLM 41102 et SLM 41103.

En appliquant le vernis à ongle selon l'invention avec le pigment SLM 41102, sur une base de vernis rouge, on obtient une nouvelle couleur orangée, avec des reflets rouge et or.

On retrouve un effet de couleurs similaires pour un vernis comprenant dans une même composition un pigment CL associé à un ou plusieurs autres pigments usuels.

### Exemple 2 Mascara transparent (il ne fait pas partie de l'invention)

| Composition | % |
|---|---|
| Acide stéarique | 6,00 |
| Glycéryl stéarate | 3,70 |
| Cire d'abeille | 5,50 |
| Cire de carnauba | 1,90 |
| Paraffine | 7,50 |
| Rosine | 1.80 |
| Ethylparaben | 0.04 |
| Propylparaben | 0.03 |
| Pigment CL | 5.00 |
| Méthylparaben | 0,23 |
| Triéthanolamine | 3,00 |
| Hydroxyéthylcellulose | 0,20 |
| Ethoxydiglycol | 0,02 |
| Accacia | 5,80 |
| Eau | qsp 100 |

On obtient un produit blanc irisé dans le flacon.

Lorsque l'on applique la composition selon l'invention avec le pigment SLM 41101 directement sur les cils noirs, on obtient un maquillage vert très intense qui devient bleu selon l'angle d'observation. On retrouve le même effet pour les couleurs spécifiques des pigments SLM 41102 et SLM 41103.

Lorsque l'on applique la composition selon l'invention sur une base de mascara noir, on obtient le même effet mais avec des couleurs plus intenses.

### Exemple 3 Mascara bleu (il ne fait pas partie se l'invention)

| Composition | % |
|---|---|
| Acide stéarique | 6,00 |
| Glycéryl stéarate | 3,70 |
| Cire d'abeille | 5,50 |
| Cire de carnauba | 1,90 |
| Paraffine | 7,50 |
| Rosine | 1,80 |
| Ethylparaben | 0,04 |
| Propylparaben | 0,03 |
| Bleu outremer | 6,10 |
| Ultramarines et silice | 0,90 |
| Dioxyde de titane | 0,50 |
| Pigment CL | 5,00 |
| Méthylparaben | 0,23 |
| Triéthanolamine | 3,00 |
| Hydroxyéthylcellulose | 0,20 |
| Ethoxydiglycol | 0,02 |
| Accacia | 5,80 |
| Eau | qsp 100 |

La couleur du mascara dans le flacon est plus intense et lumineuse que le même mascara en l'absence de pigment CL (témoin). Avec le pigment SLM 41101, la composition prend des reflets bleu vert et or.

Lorsque l'on applique la composition selon l'invention sur les cils, on obtient une coloration bleue plus intense qu'avec le témoin. De plus, le maquillage devient vert selon l'angle d'observation.

### Exemple 4 Crème de visage émulsion huile dans eau (il ne fait pas partie de l'invention)

| Composition | % |
|---|---|
| Acide stéarique | 2,00 |
| Stéarate de glycéryle | 3,00 |
| Isostéarate de glycéryle | 2,00 |
| Huile minérale | 8,00 |
| Propylparaben | 0,20 |
| Diméthicone | 4,00 |
| Pigment CL | 5,00 |
| Triéthanolamine | 1,00 |
| Méthylparaben | 0,20 |
| Magnésium aluminium silicate gel à 5 % | 20,00 |
| Gomme de cellulose | 3,50 |
| Lauroyl sarcosinate de sodium | 3,50 |
| Glycérine | 2,00 |
| Diazolildinyl urée | 0,30 |
| Eau | qsp 100 |

On obtient une crème blanche avec des reflets irisés.

Lorsque l'on applique la composition selon l'invention sur le dos de la main, on obtient un effet très lumineux, légèrement pailleté avec des reflets colorés. Lorsque l'on applique la composition selon l'invention comprenant le pigment CL SLM 41102, sur une base de rouge à lèvres rouge, on obtient une couleur plus brillante avec des reflets or. On retrouve un effet de couleurs similaires pour un rouge à lèvres comprenant dans une même composition un pigment CL associé à un ou plusieurs autres pigments usuels.

## Revendications

1. Composition cosmétique de maquillage, destinée au maquillage des ongles, comprenant un support cosmétiquement acceptable, un agent de coloration CL susceptible de produire une couleur dans une gamme de nuances comprises entre au moins deux couleurs spécifiques et variant en fonction de l'incidence de la lumière et de l'angle d'observation; ledit agent de coloration étant choisi parmi les polymères linéaires ou cycliques sur lesquels sont greffés des groupements mésomorphes; et au moins un agent de coloration non CL utilisé en combinaison avec ledit agent de coloration CL;
- la quantité totale d'agents de coloration, CL et non CL, étant comprise entre 0,1 et 30% en poids par rapport au poids total de la composition;
- le rapport pondéral agents de coloration CL / agents de coloration non CL étant compris entre 20 / 1 et 1/5.

2. Composition selon la revendication 1, dans laquelle la quantité totale d'agents de coloration, CL et non CL, est comprise entre 1 et 20% en poids par rapport au poids total de la composition.

3. Composition selon l'une des revendications précédentes, dans laquelle le rapport pondéral agents de coloration CL / agents de coloration non CL est compris entre 10/1 et 1/5.

4. Composition selon l'une des revendications précédentes, dans laquelle le rapport pondéral agents de coloration CL / agents de coloration non CL est compris entre 5/1 et 1/5.

5. Composition selon l'une des revendications précédentes, dans laquelle l'agent de coloration CL est sous forme de poudre constituée de substances orientées à réticulation tridimentionelle comprenant un groupe mésomorphe, ayant une épaisseur moyenne comprise entre 1 et 100 µm et un diamètre moyen compris entre 1 et 10 000 µm.

6. Composition selon l'une des revendications précédentes, dans laquelle l'agent de coloration non CL est choisi parmi les pigments minéraux, les pigments organiques, les laques et/ou les pigments nacrés.

7. Composition selon l'une des revendications précédentes, **caractérisée en ce que** l'agent de coloration non CL est choisi parmi les oxydes métalliques, en particulier les oxydes de zirconium, de cérium, de zinc ou de chrome, le dioxyde de titane, les oxydes de fer noir, jaune, rouge et brun, le violet de manganèse, le bleu outremer, le bleu ferrique, l'hydrate de chrome, la poudre d'argent ou la poudre d'aluminium; le noir de carbone; des sels métalliques (notamment Al, Zr, Ca, Na) de colorants organiques, adsorbés sur des particules, par exemple d'alumine, de sulfate de baryum, de colophane; l'oxychlorure de bismuth, le mica recouvert d'oxyde de titane, d'oxyde de fer, ou de pigments naturels, par exemple le mica titane coloré; les colorants organiques naturels, tels que le carmin de cochenille, les colorants organiques de synthèse tels que les colorants halogéno-acides, azoïques, anthraquinoniques; des colorants minéraux tels que le sulfate de cuivre.

## Claims

1. Cosmetic makeup composition, intended for making up the nails, comprising a cosmetically acceptable support and an LC colouring agent capable of producing a colour within a range of hues included between at least two specific colours and varying as a function of the incidence of the light and of the angle of observation; the said colouring agent being chosen from linear or cyclic polymers onto which are grafted mesomorphic groups; and at least one non-LC colouring agent used in combination with the said LC colouring agent ;
- the total amount of LC and non-LC colouring agents being between 0.1 and 30% by weight with respect to the total weight of the composition;
- the LC colouring agents/non-LC colouring agents ratio by weight being between 20/1 and 1/5.

2. Composition according to Claim 1, in which the total amount of LC and non-LC colouring agents is between 1 and 20% by weight with respect to the total weight of the composition.

3. Composition according to either of the preceding claims, in which the LC colouring agents/non-LC colouring agents ratio by weight is between 10/1 and 1/5.

4. Composition according to one of the preceding claims, in which the LC colouring agents/non-LC colouring agents ratio by weight is between 5/1 and 1/5.

5. Composition according to one of the preceding claims, in which the LC colouring agent is in the form of a powder, composed of oriented substances with three-dimensional crosslinking comprising a mesomorphic group, having a mean thickness of between 1 and 100 µm and a mean diameter of between 1 and 10 000 µm.

6. Composition according to one of the preceding claims, in which the non-LC colouring agent is chosen from inorganic pigments, organic pigments, lakes and/or pearlescent pigments.

7. Composition according to one of the preceding claims, **characterized in that** the non-LC colouring agent is chosen from metal oxides, in particular zirconium, cerium, zinc or chromium oxides, titanium dioxide, black, yellow, red and brown iron oxides, manganese violet, ultramarine blue, iron blue, chromium hydrate, silver powder or aluminium powder; carbon black; metal salts (in particular Al, Zr, Ca or Na) of organic dyes adsorbed on particles, for example of alumina, of barium sulphate or of colophony; bismuth oxychloride or mica covered with titanium oxide, with iron oxide or with natural pigments, for example coloured titanium dioxide-coated mica; natural organic dyes, such as cochineal carmine, or synthetic organic dyes, such as haloacid, azo or anthraquinone dyes; or inorganic dyes, such as copper sulphate.

## Patentansprüche

1. Kosmetische Zusammensetzung zum Schminken, die zum Schminken der Nägel dienen soll, die in einem kosmetisch akzeptablen Träger ein FK-Farbmittel, das befähigt ist, in einem zwischen mindestens zwei speziellen Farben liegenden Nuancenbereich eine Farbe zu bilden, die sich in Abhängigkeit vom Lichteinfall und dem Betrachtungswinkel verändert; wobei das Farbmittel unter den linearen oder cyclischen Polymeren ausgewählt ist, auf die mesomorphe Gruppen gepfropft sind; und mindestens ein Nicht-FK-Farbmittel enthält, das in Kombination mit dem FK-Farbmittel verwendet wird;
- wobei die Gesamtmenge der Farbmittel, FK und Nicht-FK, im Bereich von 0,1 bis 30 Gew.-%, bezogen auf das Gesamtgewicht der Zusammensetzung, liegt; und
- das Gewichtsverhältnis FK-Farbmittel/Nicht-FK-Farbmittel im Bereich von 20/1 bis 1 /5 liegt.

2. Zusammensetzung nach Anspruch 1, wobei die Gesamtmenge der Farbmittel, FK und Nicht-FK, im Bereich von 1 bis 20 Gew.-%, bezogen auf das Gesamtgewicht der Zusammensetzung, liegt.

3. Zusammensetzung nach einem der vorhergehenden Ansprüche, wobei das Gewichtsverhältnis FK-Farbmittel/Nicht-FK-Farbmittel im Bereich von 10 / bis 1/5 liegt.

4. Zusammensetzung nach einem der vorhergehenden Ansprüche, wobei das Gewichtsverhältnis FK-Farbmittel/Nicht-FK-Farbmittel im Bereich von 5 / 1 bis 1/5 liegt.

5. Zusammensetzung nach einem der vorhergehenden Ansprüche, wobei das FK-Farbmittel in Form eines Pulvers vorliegt, das aus orientierten Substanzen mit dreidimensionaler Vernetzung besteht, die eine mesomorphe Gruppe enthalten, mit einer mittleren Dicke von 1 bis 100 µm und einem mittleren Durchmesser von 1 bis 10 000 µm.

6. Zusammensetzung nach einem der vorhergehenden Ansprüche, wobei das Nicht-FK-Farbmittel unter den anorganischen Pigmenten, organischen Pigmenten, Lacken und/oder Perlglanzpigmenten ausgewählt ist.

7. Zusammensetzung nach einem der vorhergehenden Ansprüche, **dadurch gekennzeichnet, dass** das Nicht-FK-Farbmittel unter den Metalloxiden und insbesondere den Oxiden von Zirconium, Cer, Zink oder Chrom, Titandioxid, schwarzem, gelbem, rotem und brauem Eisenoxid, Manganviolett, Ultramarinblau, Eisenblau, Chromhydrat, Silberpulver oder Aluminiumpulver; Ruß; Metallsalzen (insbesondere Al, Zr, Ca, Na) von organischen Farbstoffen, die auf Partikeln absorbiert sind, wie beispielsweise Aluminiumoxid, Bariumsulfat, Kolophonium; Bismutoxidchlorid, mit Titanoxid, Eisenoxid oder natürlichen Pigmenten bedeckten Glimmerpigmenten, beispielsweise farbigen Titandioxid-Glimmer-Pigmenten; natürlichen organischen Farbstoffen, wie Cochenille-Karmin, synthetischen organischen Farbstoffen, wie Halogenosäuren, Azofarbstoffen, Anthrachinon-Farbstoffen; anorganischen Farbmitteln, wie Kupfersulfat, ausgewählt ist.
